# EUROPÄISCHE PATENTSCHRIFT

(11) **EP 0 702 003 B1**
(45) Veröffentlichungstag und Bekanntmachung des Hinweises auf die Patenterteilung: **17.06.1998**
(21) Anmeldenummer: 95112442.9
(22) Anmeldetag: 08.08.1995
(51) Int. Cl.: C07D 213/61, C07D 213/65, C07D 213/78, C07D 213/85

(54) **Verfahren zur Herstellung von 2-Halogenpyridinaldehyden**
Process for preparing 2-halogenpyridinaldehydes
Procédé pour la préparation de 2-halogenpyridinaldehydes

(30) Priorität: 19.08.1994 DE 4429465
(43) Veröffentlichungstag der Anmeldung: 20.03.1996
(73) Patentinhaber: BAYER AG, 51368 Leverkusen (DE)
(72) Erfinder: Beck, Gunther, Dr., D-51375 Leverkusen (DE); Heitzer, Helmut, Dr., D-51381 Leverkusen (DE)

(56) Entgegenhaltungen:
- WO-A-95/15313
- IND. JOURN. CHEM., Bd. 28B, 1989 Seiten 584-586, SREENIVASULU ET AL. 'A one-pot synthesis of ...'
- SYNTHETIC COMMUNICATIONS, Bd. 23, Nr. 18, 1993 Seiten 2587-2592, AADIL ET AL. 'Synthesis of 4,5 disubstituted 2-chloronicotinates'
- J. CHEM. SOC., PERKIN TRANS. 1 (JCPRB4,0300922X);84; (6); PP.1173-82, UNIV. SALFORD;CHEM. DEP.; SALFORD; MS 4WT; UK (GB), METH-COHN O ET AL 'A versatile new synthesis of quinolines and related fused pyridines. Part 12. A general synthesis of 2-chloropyridines and 2-pyridones'
- J. ORG. CHEM., Bd. 55, 1990 Seiten 5793-5797, GUZMAN ET AL. 'Vilsmeier-Haack Reaction with ...'
- J. ORGANOMETALLIC CHEMISTRY, Bd. 406, 1991 Seiten 49-56, MALLET 'Le phényl-lithium: nouvel angent de métalisation du cycle pryridinique'
- SYNTHESIS, Januar 1994 Seiten 87-92, WINDSCHEIF 'Substituted dipyridylethenes ...'
- J. MED. CHEM., Bd. 31, 1988 Seiten 618-624, NEW ET AL. 'Atypical antipsychotic agents: ...'
- C.A.120:134231
- C.A.26:5096

## Beschreibung

Die Erfindung betrifft ein Verfahren zur Herstellung von 2-Halogenpyridinen, die in 3- und/oder 5-Position Aldehydgruppen tragen, durch Umsetzung von Cyanolefinen, Aminocarbonylolefinen oder Hydroxyiminoolefinen mit einem Vilsmeier-Reagens.

Die wenigen bisher bekannten 2-Halogenpyridinaldehyde mußten entweder durch vielstufige Synthesen hergestellt werden oder sind als Nebenprodukte in nur geringen Ausbeuten angefallen (z.B. US-PS 4 279 913; J. Org. Chem. 55 (1990), 5793; J. Chem. Soc., Perkins Trans. I 1984, 1173). Da sie als Zwischenprodukte für die Herstellung von Pharmazeutika dienen können (US-PS 4 279 913), bestand ein Bedarf nach einem wirtschaftlich sinnvollen Herstellungsverfahren.

Überraschenderweise wurde gefunden, daß die gewünschten Produkte über eine Reaktion mit Vilsmeier-Reagens zugänglich sind.

Gegenstand der Erfindung ist also ein Verfahren zur Herstellung von Verbindungen der Formel (I) 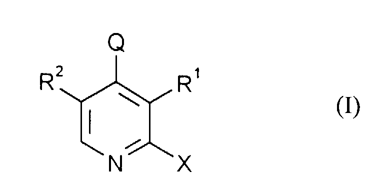 worin
- X: Chlor oder Brom,
- Q: Wasserstoff, Halogen, C₆-C₁₂-Aryl, Hetaryl mit 5 bis 7 Ringgliedern und 1 bis 3 Heteroatomen aus der Reihe N, O, S,
- R¹, R²: Formyl, Cyan, Hydroxyl, Halogen, C₁-C₈-Alkyl, Halogen-C₁-C₈-alkyl, Cyan-C₁-C₈-alkyl, C₁-C₄-Alkoxy-C₁-C₈-alkyl, C₃-C₁₀-Cycloalkyl, 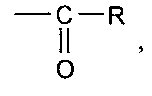 C₆-C₁₂-Aralkyl, C₆-C₁₂-Aryl, C₆-C₁₂-Aryloxy, Hetaryl mit 5 bis 7 Ringgliedern und 1 bis 3 Heteroatomen aus der Reihe N, O, S, Hetaryloxy mit 5 bis 7 Ringgliedem und 1 bis 3 Heteroatomen aus der Reihe N, O, S im Hetarylteil,
mit der Maßgabe, daß mindestens einer der beiden Substituenten R¹ und R² für Formyl steht,
- R: Halogen, C₁-C₄-Alkoxy, Phenoxy, -NR'R" und
- R', R": unabhängig voneinander C₁-C₄-Alkyl oder beide Substituenten zusammen C₄-C₆-Alkylen, das durch ein Sauerstoffatom unterbrochen sein kann, bedeuten,
dadurch gekennzeichnet, daß man eine Verbindung der Formel (II) 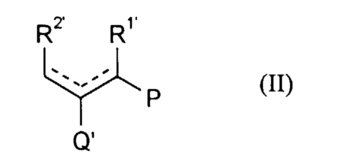 worin
die gestrichelte Linie die beiden möglichen Positionen einer C-C-Bindung anzeigt,
- Q': die für Q definierten Bedeutungen annehmen kann oder Hydroxyl oder C₁-C₄-Alkoxy bedeutet,
- P: -CN, -CONH₂ oder -CH=NOH und
- R^{1'}, R²': unabhängig voneinander Wasserstoff, Cyan, Halogen, -CONH₂, C₁-C₈-Alkyl, Halogen-C₁-C₈-alkyl, Cyan-C₁-C₈-alkyl, C₁ -C₄-Alkoxy-C₁-C₈-alkyl, Hydroxy-C₁-C₈-alkyl, C₁-C₈-Alkoxy, C₃-C₁₀-Cycloalkyl, 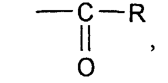 Carboxyl, C₁-C₈-Acyloxy, C₆-C₁₂-Aralkyl, C₆-C₁₂-Aryl, C₆-C₁₂-Aryloxy, Hetaryl mit 5 bis 7 Ringgliedern und 1 bis 3 Heteroatomen aus der Reihe N, O, S, Hetaryloxy mit 5 bis 7 Ringgliedern und 1 bis 3 Heteroatomen aus der Reihe N, O, S im Hetarylteil mit der Maßgabe, daß mindestens einer der beiden Substituenten R^{1'} und R^{2'} für Wasserstoff steht, bedeuten und
- R: die in Formel (I) angegebene Bedeutung besitzt,
mit einem Vilsmeier-Reagens umsetzt.

In den obigen Definitionen bedeuten vorzugsweise
- "Alkyl": C₁-C₄-Alkyl wie Methyl, Ethyl,
- "Halogen": Fluor, Chlor, Brom, insbesondere Chlor,
- "Halogenalkyl": Halogen-C₁-C₄-alkyl wie Chlormethyl, 1- und 2-Chlorethyl, Di- und Trifluormethyl,
- "Cyanalkyl": Cyan-C₁-C₄-alkyl wie Cyanmethyl,
- "Alkoxyalkyl": C₁ -C₄-Alkoxy-C₁-C₄-alkyl wie Methoxymethyl,
- "Hydroxyalkyl": Hydroxy-C₁-C₄-alkyl wie Hydroxymethyl, 1- und 2-Hydroxyethyl,
- "Alkoxy": C₁-C₄-Alkoxy wie Methoxy, Ethoxy,
- "Cycloalkyl": C₅-C₇-Cycloalkyl wie Cyclopentyl, Cyclohexyl und Cycloheptyl,
- R: Methoxy, Ethoxy und von sekundären aliphatischen Aminen abgeleitete Aminogruppen wie Dimethylamino, Diethylamino, Pyrrolidino, Piperidino, Morpholino, Hexamethylenimino,
- "Acyloxy": C₂-C₇-Acyloxy, insbesondere aliphatisches C₂-C₄-Acyloxy, vor allem Acetyl, oder Benzoyl,
- "Aralkyl": C₇-C₁₂-Aralkyl wie Benzyl, 1- und 2-Phenethyl,
- "Aryl": C₆-C₁₀-Aryl wie Phenyl, Naphthyl,
- "Aryloxy": C₆-C₁₀-Aryloxy wie Phenoxy, Naphthoxy,
- "Hetaryl": 5- und 6-gliedrige Ringsysteme mit 1 oder 2 Heteroatomen aus der Reihe N, O, S, wie 2-, 3- und 4-Pyridyl, 2- und 3-Thienyl, 2-, 4- und 5-Thiazolyl, 2-Benzthiazolyl, 2-, 4- und 5-Pyrimidyl, wobei die genannten Ringe 1- oder 2-fach substituiert sein können (z.B. durch Halogen, Methyl, Methoxy),
- "Hetaryloxy": die den "Hetaryl"-Resten analogen Reste.

Die Ausgangsverbindungen (II) umfassen z.B.
a) Crotononitrile der Formel (IIa) 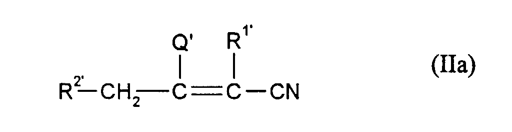
b) Allylcyanide der Formel (IIb) 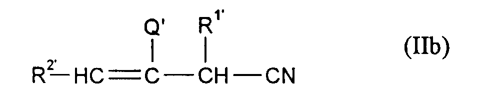
c) Crotonsäureamide der Formel (IIc) 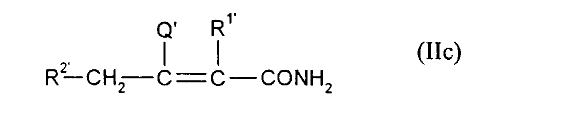
d) But-3-enamide der Formel 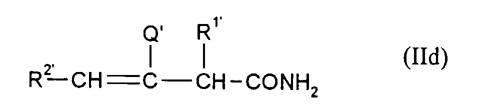
e) Oxime von Crotonaldehyden der Formel 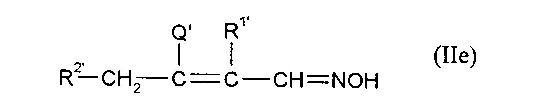 und
f) Oxime von Vinylacetaldehyden der Formel

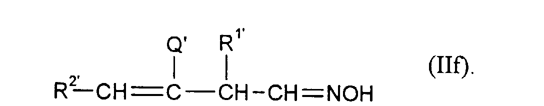

Die Verbindungen (II) sind bekannt oder können analog bekannten Verfahren hergestellt werden.

Nachfolgend werden bevorzugte Ausgangsverbindungen (IIa) und (IIb) aufgezählt, womit dann auch die analogen Verbindungen (IIc) bis (IIf) erfaßt sein sollen. Der besseren Übersichtlichkeit wegen werden die Ausgangsverbindungen entsprechend den gewünschten Endprodukten in 3 Gruppen untergliedert.
1. Ausgangsstoffe für 2-Halogen-3-pyridincarboxaldehyde:
   NC-CH₂-CH=CH-CN, Cl-CH₂-CH₂-CH=CH-CN, Cl-CH₂-CH=CH-CN, 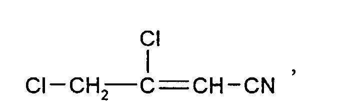 NC-CH=CH-CH₂-C₂H₅ , 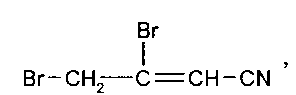 CH₃O-CH₂-CH=CH-CN, CH₃-CH=CH-CH₂-CN, CH₃-CH₂-CH=CH-CN, HO-CH₂-CH=CH-CH₂-CN, Cl-CH₂-CH=CH-CH₂-CN, NC-CH₂-CH=CH-CH₂-CN, NC-CH=CH-CH₂-CH₂-CN, 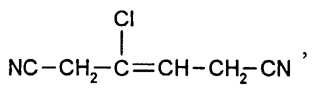
   CH₃-CO-OCH₂-CH=CH-CN, CH₃-CO-O-CH=CH-CH₂-CN, NC-CH=CH-CH₂-COOC₂H₅, NC-CH₂-CH=CH-COOC₂H₅, NC-CH₂-CH=CH-CH₂-CH₂-OCH₃, Phenyl-CH=CH-CH₂-CN, Phenyl-CH₂-CH=CH-CN, Phenyl-CH₂-CH=CH-CH₂-CN, 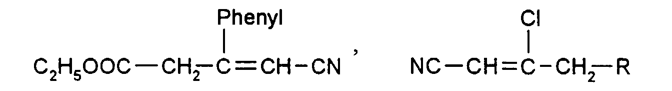 (R=CH₃, C₂H₅, n-C₃H₇, i-C₃H₇, n-C₄H₉,n-C₆-H₁₃, -CH₂-Phenyl, Phenyl), 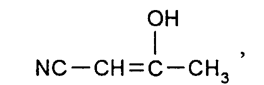 Cl-CH=CH-CH₂-CN.
2. Ausgangsstoffe für 2-Halogen-5-pyridincarboxaldehyde: 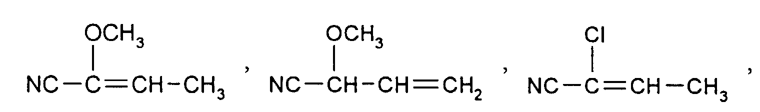 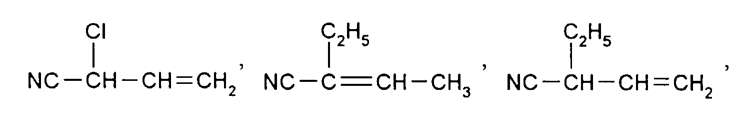 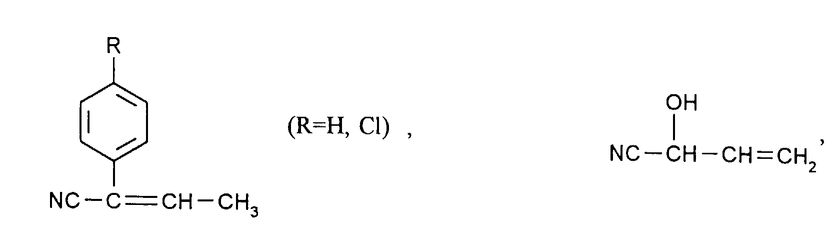 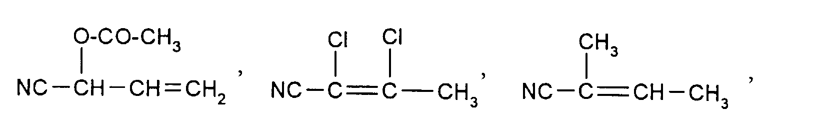 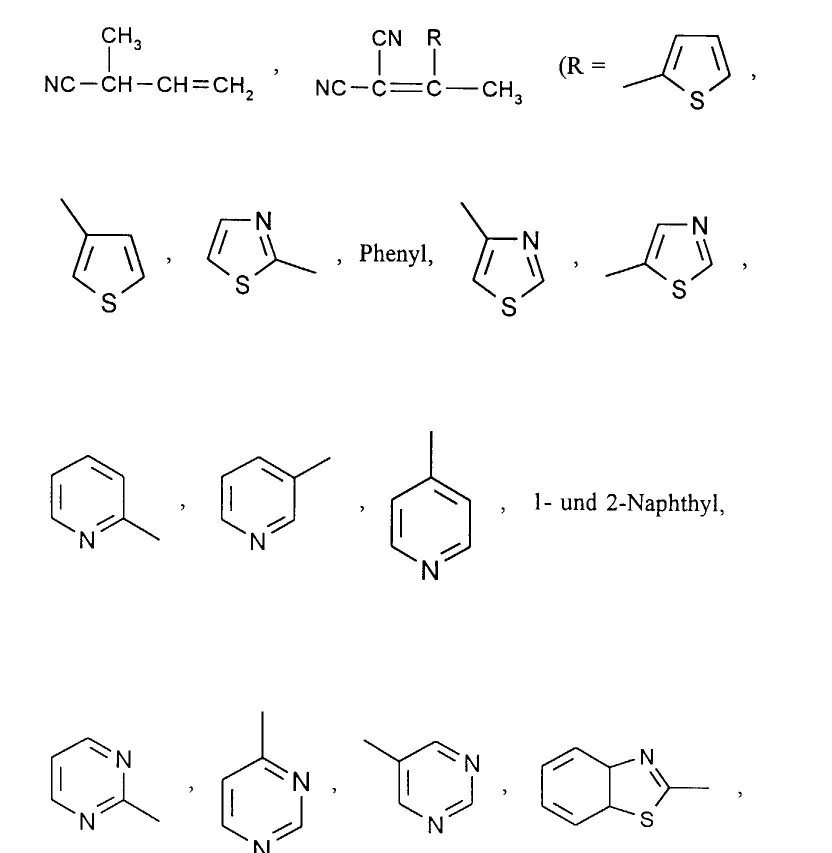 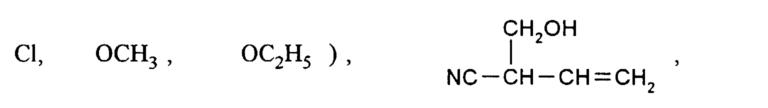 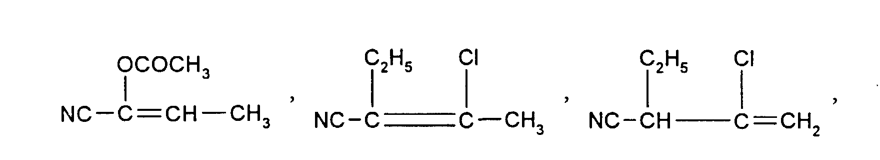 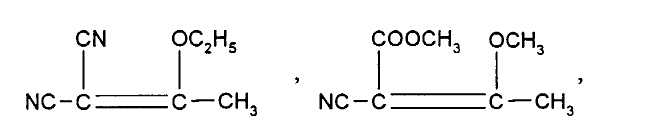 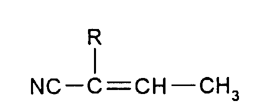 (R= CN, COOCH₃ , COOC₂H₅, COOH), 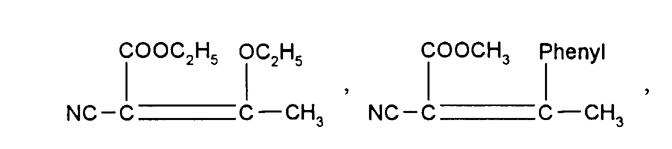 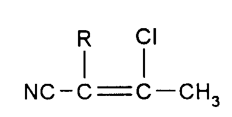 (R = CH₃ , C₂H₅, CH₂CH₂Cl , CH₂CH₂-O-CO-CH₃, OCH₃, n-C₃H₇, i-C₃H₇, n-C₄H₉, n-C₆H₁₃, CH₂-Phenyl, Phenyl), 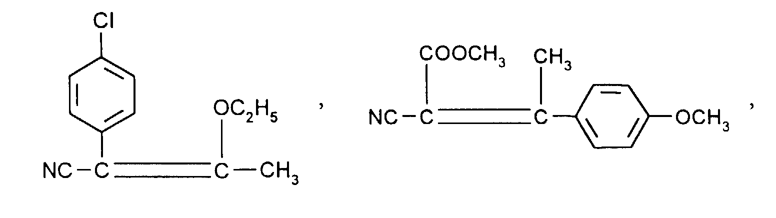 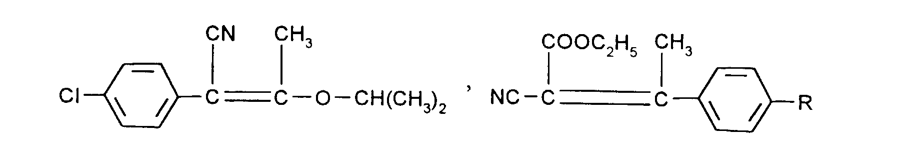 (R = H, Cl, CH₃, OCH₃, Br, F), 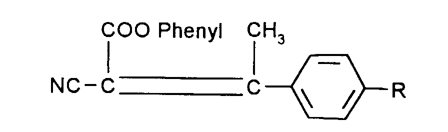 (R = H, F, Cl, Br, CH₃, OCH₃); 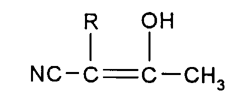 (R = H, Phenyl, Cl, CN. COOCH₃, COOC₂H₅).
3. Ausgangsstoffe für 2-Halogen-3,5-pyridindicarboxaldehyde:
   CH₃-CH=CH-CN, CH₂=CH-CH₂-CN, 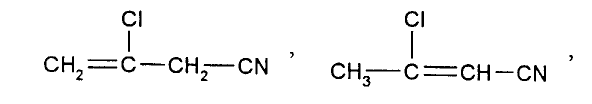 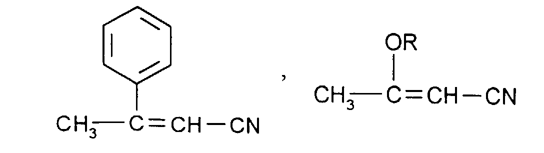 (R = CH₃ , C₂H₅).
   Besonders bevorzugte Ausgangsverbindungen (II) sind solche, bei denen Q' Wasserstoff oder Chlor bedeutet.
Als Vilsmeier-Reagens kommen die bekannten Umsetzungsprodukte von N-Formylamiden mit Säurehalogeniden in Betracht. Als N-Formylamide seien beispielsweise genannt: Dimethylformamid, N-Methylformanilid, N-Formylmorpholin. Als Säurehalogenide seien beispielsweise genannt: Phosphoroxychlorid, Phosphoroxybromid, Phosgen, Thionylchlorid, Oxalylchlorid. Als Lösungsmittel können Dimethylformamid, Chloralkane, Chloralkene, Chlorbenzole oder Phosphoroxychlorid oder ein Überschuß des (z.B. aus Phosphoroxychlorid und Dimethylformamid erzeugten) Vilsmeier-Reagens verwendet werden (vergleiche hierzu: Advances in Heterocyclic Chemistry, Vol. 31 (1982), Seite 207).

Die Umsetzung der Verbindungen (II) mit dem Vilsmeier-Reagens kann unter allgemein bekannten Bedingungen erfolgen. Vorzugsweise werden pro Mol der Verbindung (II) für den Fall, daß R^{1'} oder R^{2'} = H und P = CN, mindestens 2 Mol Vilsmeier-Reagens und für den Fall, daß R^{1'} und R^{2'} = H und P = CN, mindestens 3 Mol Vilsmeier-Reagens eingesetzt. Die Synthese des Dialdehyds 2-Chlor-3,5-pyridindicarboxaldehyd zeigt jedoch, daß auch mit einem Unterschuß an Vilsmeier-Reagens, z.B. selbst mit 10 % der stöchiometrisch erforderlichen Menge, die gewünschten Aldehyde I gebildet werden (Beispiel 4).

Zur Erzielung einer möglichst hohen Ausbeute an 2-Halogenpyridinaldehyden(I) kann es besonders vorteilhaft sein, einen Überschuß an Vilsmeier-Reagens einzusetzen, der bis 500 % der stöchiometrisch erforderlichen Menge betragen kann. Eine Erhöhung der Reaktionszeit und eine Erhöhung der Reaktionstemperatur bewirken im allgemeinen eine Erhöhung der Ausbeute. Bevorzugt sind Reaktionstemperaturen von 0 bis 180, vorzugsweise 60 bis 160, insbesondere 90 bis 110°C und Reaktionszeiten von etwa 24 Stunden, doch können noch höhere Reaktionszeiten bis zu etwa 100 Stunden von Vorteil sein.

Im allgemeinen werden bei der Durchführung des erfindungsgemäßen Verfahrens N-Formylamid, Säurehalogenid und Ausgangsstoff (I) bei Temperaturen zwischen etwa 0 und 5°C vereinigt, gegebenenfalls eine Zeitlang in diesem Temperaturbereich nachgerührt, denn allmählich (z.B. im Verlauf einer Stunde) auf die gewünschte Endtemperatur gebracht und bei dieser Temperatur noch die gewünschte Reaktionszeit gehalten.

Es ist jedoch auch möglich, das erfindungsgemäße Verfahren von Beginn an bei der gewünschten Endtemperatur durchzuführen. Hierbei hat sich folgende Methode als besonders einfach erwiesen: Man tropft zum auf die Endtemperatur (z.B. 95 bis 100°C) vorgeheizten Säurehalogenid (vorzugsweise Phosphoroxychlorid) die Lösung des Ausgangsstoffs in dem N-Formylamid (vorzugsweise Dimethylformamid). Diese Verfahrensvariante ist wegen ihrer bequemen technischen Handhabung besonders bevorzugt.

Während des erfindungsgemäßen Verfahrens oder der anschließenden wäßrigen Aufarbeitung des Reaktionsgemisches werden die unter diesen Bedingungen nicht inerten Substituenten verändert oder ausgetauscht, und zwar entstehen in der Regel aus

| | |
|---|---|
| -CONH₂ | Nitril, |
| Hydroxyl | Halogen (also |
| Hydroxyalkyl | Halogenalkyl), |
| Acyloxy | Hydroxyl und |
| Carboxyl | COCl. |

Alkoxy, insbesondere Methoxy, kann durch Halogen substituiert werden.

Darüberhinaus können COCl-Gruppen durch die Einwirkung des Vilsmeier-Reagens teilweise oder vollständig in die entsprechenden Carbonamide überführt werden. So kann also bei Einsatz von Dimethylformamid aus COOH in (II) CON(CH₃)₂ in (I) entstehen.

Aktivierte Methylengruppen, z.B. NC-CH₂-Gruppen, können durch die Einwirkung des Vilsmeier-Reagens teilweise oder vollständig weiterreagieren, so daß bei Einsatz von Dimethylformamid aus NC-CH₂ in (II) 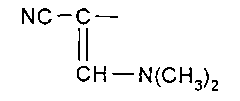 in (I) entstehen kann.

Das erfindungsgemäße Verfahren ist durch den Stand der Technik nicht nahegelegt, sondern ist außerordentlich überraschend:

In Indian Journal of Chemistry, Vol. 28 B, Seite 584 (1989) ist beschrieben, daß Alkylidenmalononitrile mit Vilsmeier-Reagens in 2-Chlor-3-cyanpyridine übergeführt werden können:

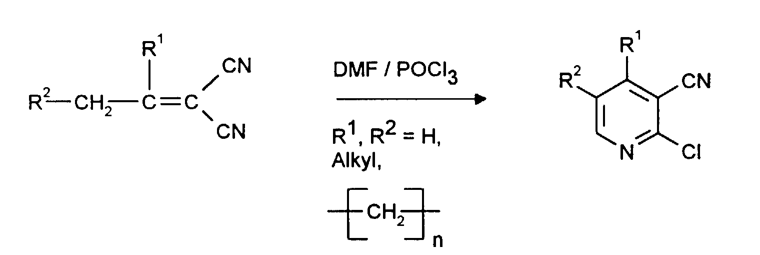

Das Schema sagt aus, daß im Falle von R¹ = R² = H 2-Chlor-3-cyanpyridin 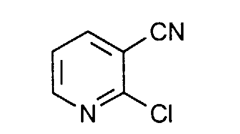 entsteht. Stattdessen wird erfindungsgemäß 2-Chlor-3-cyan-5-pyridincarboxaldehyd 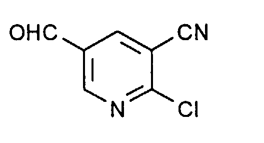 gebildet. Ersetzt man eine der beiden Cyangruppen in den oben formulierten Alkylidenmalononitrilen durch Wasserstoff, so mußte man - falls wegen der fehlenden zweiten aktivierenden Nitrilgruppen überhaupt noch eine Umsetzung erfolgt - eine Verbindung der Formel 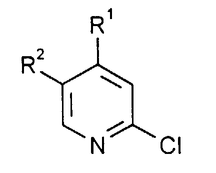 erwarten. Stattdessen entsteht erfindungsgemäß beispielsweise für den Fall R¹ = H, R² = CH₃ der 2-Chlor-5-methyl-3-carboxaldehyd 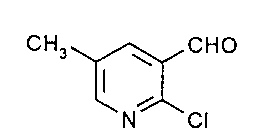 und beispielsweise für den Fall R¹ = R² = H der 2-Chlor-3,5-pyridindicarboxaldehyd 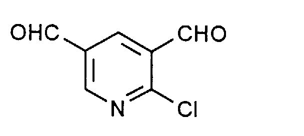 Wir konnten übrigens nachweisen, daß aus 2-Chlorpyridin unter den Bedingungen des erfindungsgemäßen Verfahrens keine Spur eines Aldehyds entsteht.

In Synthetic Communications, Vol. 23, Seite 2587 (1993) ist in Analogie zu der oben zitierten Arbeit beschrieben, daß Alkylidencyanessigsäureester mit Vilsmeier-Reagens in 2-Chlor-3-pyridincarbonsäureester übergeführt werden können: 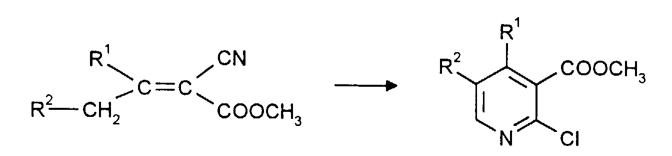 In der Arbeit wird behauptet, daß im Falle von R² = H kein Ringschluß stattfindet.

Es war daher nicht vorhersehbar, daß stattdessen erfindungsgemäß beispielsweise für den Fall R¹ = R² = H der 2-Chlor-5-formyl-3-pyridincarbonsäureester 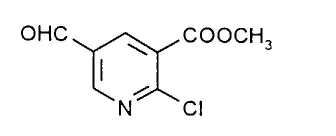 gebildet wird.

Die meisten der Verbindungen I sind neu.

Bevorzugte Verbindungen I entsprechen der Formel (III) 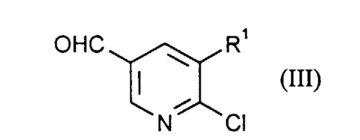 worin
- R¹: für Hydroxy, Cyan, Methyl, Phenyl, Chlormethyl, Chlorcarbonyl, Methoxyoder Ethoxycarbonyl steht,
der Formel 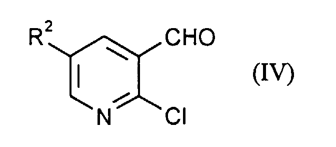 worin
- R²: für Hydroxy, Cyan, Chlor, Phenyl, Chlormethyl, Chlorcarbonyl, Methoxyoder Ethoxycarbonyl steht,
der Formel 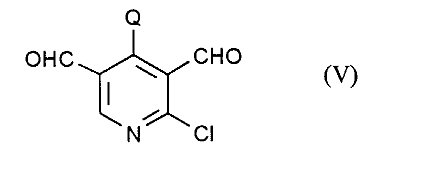 worin
- Q: für Chlor oder Phenyl steht,
oder der Formel 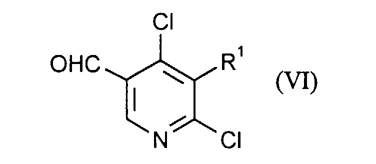 worin
- R¹: für Cyan oder Phenyl steht.

Die Verbindungen I können als Zwischenprodukte zur Herstellung von Pharmazeutika und Pflanzenschutzmitteln verwendet werden; vergleiche allgemein K. H. Büchel (Herausg.), Pflanzenschutz und Schädlingsbekämpfung, Georg Thieme Verlag, Stuttgart 1977, Stoffklasse "Pyridine" (S. 229) und insbesondere US-PS 4 757 073, DE-OS 24 27 096, 33 14 196, EP-A 1473, 107 866 und 192 060, JP-A 05/17 475 (26. 1. 1993) und 04/128 284 (28. 4. 1992).

Die Prozentangaben der nachfolgenden Beispiele beziehen sich jeweils auf das Gewicht.

### Beispiele

### Beispiel 1

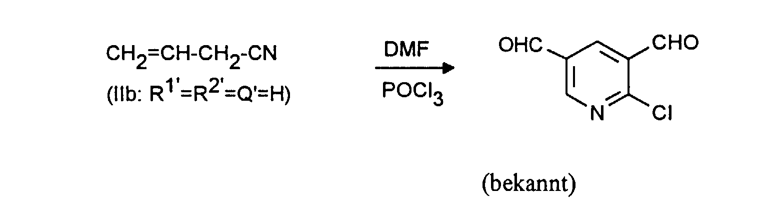

Zu 307 g (2 Mol) Phosphoroxychlorid wird unter Rühren und unter Feuchtigkeitsausschluß bei 95 bis 100°C im Verlauf einer Stunde eine Mischung aus 26,8 g (0,4 Mol) Allylcyanid und 146 g (2 Mol) Dimethylformamid (DMF) getropft. Es wird 24 Stunden bei 95 bis 100°C nachgerührt. Nach dem Abkühlen auf etwa 20°C wird zuerst mit 500 ml Dichlormethan verrührt und anschließend 500 ml Wasser unter Kühlung so zugegeben, daß die Innentemperatur 25°C nicht übersteigt. Die organische Phase wird abgetrennt und die wäßrige Phase noch dreimal mit je 250 ml Dichlormethan extrahiert. Die vereinigten Dichlormethan-Phasen werden zweimal mit je 500 ml Wasser gewaschen, über Natriumsulfat getrocknet und im Rotationsverdampfer eingeengt. Es hinterbleiben 34,2 g (50,4 % der Theorie) 2-Chlor-3,5-pyridindicarboxaldehyd mit einer GC-Reinheit von 98 %. Nach dem Umkristallisieren aus Cyclohexan lange dünne Nadeln vom Schmelzpunkt 77 bis 78°C. In J. Org. Chem. 55 (1990), 5793 wird ein Schmelzpunkt von 70 bis 71°C angegeben; die dort zitierten IR- und ¹H-NMR-Daten stimmen mit den hier gefundenen überein.

### Beispiel 2

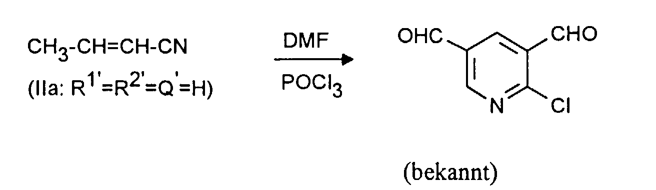

Man verfährt analog Beispiel 1 mit dem Unterschied, daß man statt 26,8 (0,4 Mol) Allylcyanid 26,8 g (0,4 Mol) Crotononitril einsetzt. Man erhält in praktisch identischer Ausbeute und Reinheit 2-Chlor-3,5-pyridindicarboxaldehyd.

### Beispiel 3

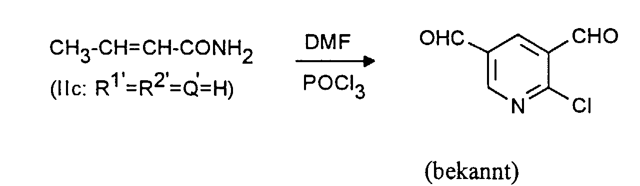

Man verfährt analog Beispiel 1 mit dem Unterschied, daß man statt 26,8 g (0,4 Mol) Allylcyanid 34 g (0,4 Mol) Crotonsäureamid einsetzt. Man erhält 2-Chlor-3,5-pyridindicarboxaldehyd in praktisch identischer Ausbeute und Reinheit.

### Beispiel 4

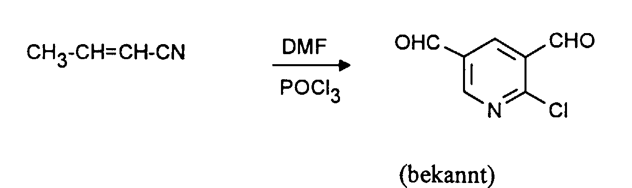

Dieses Beispiel demonstriert, daß auch mit einem Unterschuß an Vilsmeier-Reagens der Dialdehyd gebildet wird:

Man verfährt analog Beispiel 1 mit dem Unterschied, daß man statt 26,8 g (0,4 Mol) Allylcyanid 26,8 g (0,4 Mol) Crotononitril und statt 146 g (2 Mol) DMF nur 40,9 g (0,56 Mol entsprechend ca. 47 % der stöchiometrisch erforderlichen Menge) DMF einsetzt. Nach dreistündigem Nachrühren bei etwa 100°C wird auf 25°C abgekühlt und das überschüssige Phosphoroxychlorid im Vakuum bei einer Badtemperatur von 25 °C abgezogen. Die weitere Aufarbeitung erfolgt analog Beispiel 1. Man erhält 12,8 g eines Substanzgemischs, das nach GC/MS-Analyse zu etwa 35 % aus Ausgangsprodukt, zu etwa 14 % aus 3-Chlorbutyronitril (HCl-Anlagerungsprodukt an Crotononitril) und zu 45 % aus 2-Chlor-3,5-pyridindicarboxaldehyd besteht. Die beiden Monoaldehyde 2-Chlor-3-pyridincarboxaldehyd und 2-Chlor-5-pyridincarboxaldehyd wurden mit 1,3 % bzw. 0,5 % nachgewiesen. 2-Chlorpyridin wurde nicht gefunden.

### Beispiel 5

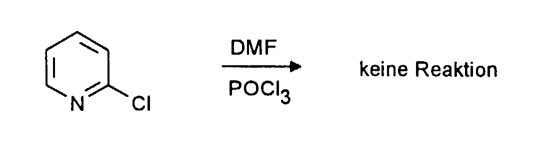

Dieses Beispiel demonstriert, daß die Synthese von 2-Chlor-3,5-pyridindicarboxaldehyd aus Allylcyanid oder aus Crotononitril nicht über die Zwischenstufen 2-Chlorpyridin läuft:

Man verfährt analog Beispiel 1 mit dem Unterschied, daß man statt 26,8 g (0,4 Mol) Allylcyanid 45,4 g (0,4 Mol) 2-Chlorpyridin einsetzt. Das erhaltene Rohprodukt enthält laut GC/MS-Analyse keine Spur eines 2-Chlorpyridin-mono- oder -dialdehyds.

### Beispiel 6

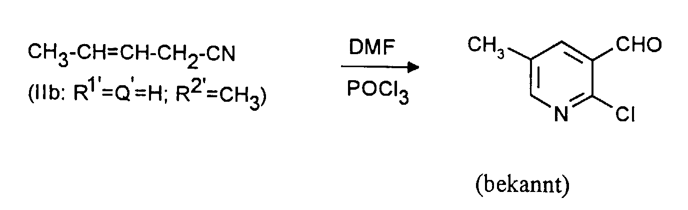

Man verfährt analog Beispiel 1 mit dem Unterschied, daß man statt 26,8 g (0,4 Mol) Allylcyanid 72,9 g (0,9 Mol) trans-Pent-3-ennitril einsetzt. Man erhält 65,6 g eines Rohprodukts, das laut GC-MS-Analyse zu 72,5 % (entsprechend 34 % der Theorie) aus 2-Chlor-5-methyl-3-pyridincarboxaldehyd besteht. Nach dem Umkristallisieren aus Cyclohexan Nadeln vom Schmelzpunkt 114 bis 114,5°C. In J. Chem. Soc., Perkin Trans. I 1984, 1173 wird ein Schmelzpunkt von 114 bis 115°C angegeben; die dort zitierten IR- und ¹H-NMR-Daten stimmen mit den hier gefundenen überein.

### Beispiel 7

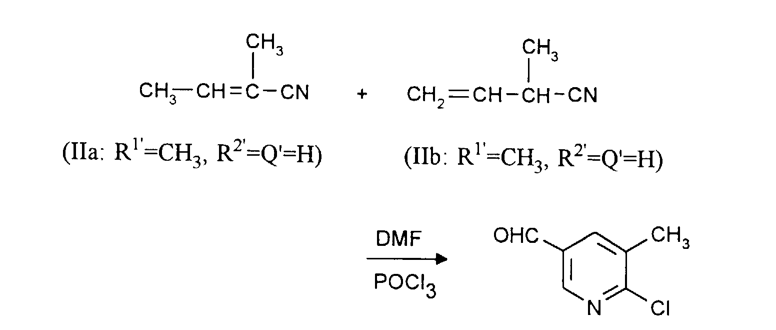

Man verfährt analog Beispiel 1 mit dem Unterschied, daß man statt 26,8 g (0,4 Mol) Allylcyanid 72,9 g (ca. 0,84 Mol) eines Substanzgemischs einsetzt, das laut ¹H-NMR-Analyse zu 54,3 Molprozent aus 2-Methyl-but-2-ennitril, zu 38,7 Molprozent aus 2-Methyl-but-3-ennitril und zu 7,0 Molprozent aus Cyclohexan besteht (Firma Fluka). Die Dichlormethanphase wird i. Vak. am Rotationsverdampfer bei einer Badtemperatur von zum Schluß 60°C eingeengt; es hinterbleiben 44,1 g eines Öls, das beim Erkalten erstarrt und das laut GC/MS-Analyse zu 61,2 % (entsprechend 20,7 % der Theorie) aus 2-Chlor-3-methyl-5-pyridincarboxaldehyd und zu etwa 24 % aus leichtflüchtigen Komponenten, darunter die oben formulierten Ausgangsstoffe, besteht. Letztere werden bei ca. 0,1 mbar und einer Badtemperatur von 60 bis 70°C abgezogen, und der erkaltete Rückstand wird aus Petrolether umkristallisiert. Schmelzpunkt 54°C.

| ¹H-NMR (CDCl₃) | |
|---|---|
| H | δ (ppm) |
| H an C-4 | 8,05 (d) - J 4,6 ≈ 2 Hz |
| H an C-6 | 8,72 (d) - J 4,6 ≈ 2 Hz |
| CHO | 10,10 (s) |
| CH₃ | 2,50 (s) |

### Beispiel 8

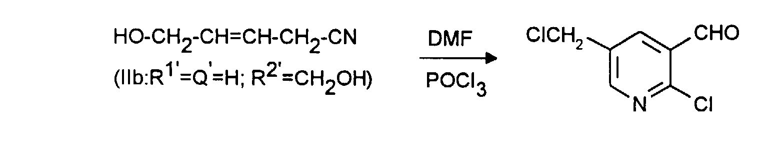

Zu 307 g (2 Mol) Phosphoroxychlorid wird unter Rühren und unter Feuchtigkeitsausschluß unter Eisbadkühlung bei 0 bis 5°C im Verlauf von etwa 30 Minuten eine Mischung aus 19,4 g (0,2 Mol) 5-Hydroxy-trans-pent-3-ennitril und 32,1 g (0,44 Mol) DMF getropft. Anschließend wird während einer Stunde bis auf 100°C erhitzt und dort noch weitere 3 Stunden gehalten. Dann wird abgekühlt und das überschüssige POCl₃ im Vakuum bei einer Badtemperatur von 25°C abgezogen. Die weitere Aufarbeitung erfolgt analog Beispiel 1. Man erhält 19,8 g eines Öls, das nach GC/MS-Analyse zu 17,7 % aus 5-Chlor-3-pentennitril und zu 74,4 % aus 2-Chlor-5-chlormethyl-3-pyridincarboxaldehyd (entsprechend 45,6 % der Theorie, bezogen auf umgesetztes Ausgangsprodukt) besteht. Fraktionierte Destillation liefert bei 101°C/0,15 mbar einen laut GC 97,8 %igen 2-Chlor-5-chlormethyl-3-pyridincarboxaldehyd, der beim Abkühlen erstarrt. Schmelzpunkt 45,5 bis 46°C nach Umkristallisieren aus Petrolether (Kp. 30-50°C).

| ¹H-NMR (CDCl₃) | |
|---|---|
| H | δ (ppm) |
| H an C-4 | 8,27 (d) |
| H an C-6 | 8,65 (d) |
| CHO | 10,45 (s) |
| CH₂Cl | 4,66 (s) |

### Beispiel 9

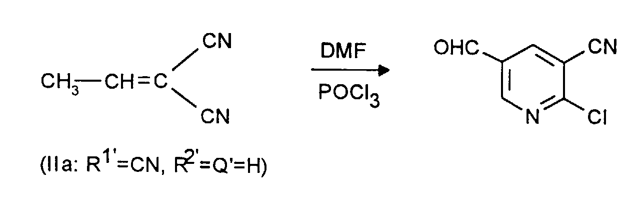

Zu 614 g (4 Mol) Phosphoroxychlorid wird unter Rühren und unter Feuchtigkeitsausschluß unter Eisbadkühlung bei 20 bis 25°C im Verlauf von 40 Minuten eine Mischung aus 292 g (4 Mol) DMF und 102 g (0,887 Mol) rohes, laut GC-Analyse 80 %iges (Rest ist Benzol) 2-Cyan-but-2-ennitril getropft. Anschließend wird während einer Stunde bis auf 95 bis 100°C aufgeheizt und dort noch weitere 24 Stunden gehalten. Nach dem Abkühlen wird entsprechend Beispiel 1 (unter jeweiliger Verdoppelung der dort angegebenen Dichlormethanund Wassermengen) aufgearbeitet. Man erhält 24,2 g (16,4 % der Theorie) 2-Chlor -3-cyan-5-pyridincarboxaldehyd als kristallines Produkt in einer GC-Reinheit von 95,2 %. Schmelzpunkt 96°C nach Umkristallisieren aus Cyclohexan.

Das 2-Cyan-but-2-ennitril wurde, ausgehend von 1 Mol Malodinitril und 1,4 Mol Acetaldehyd in 1000 ml Benzol unter Zusatz von 0,1 ml Diethylamin nach den Angaben in Can. J. Chem. 48, 773 (1965) hergestellt, jedoch wegen seiner bekannten hohen Neigung zur Trimerisation [vergleiche Can. J. Chem. 52, 3626 (1974)] nicht destilliert, sondern als Rohware eingesetzt. Ausbeute an 2-Chlor-3-cyan-5-pyridincarboxaldehyd über beide Stufen, bezogen auf 1 Mol Malodinitril: 14,5 % der Theorie.

| ¹H-NMR (CDCl₃) | |
|---|---|
| H | δ (ppm) |
| H an C-4 | 8,48 (d) |
| H an C-6 | 9,05 (d) |
| CHO | 10,15 (s) |

### Beispiel 10

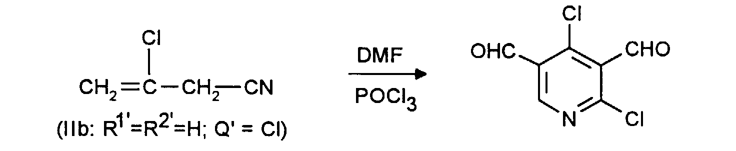

Man verfährt analog Beispiel 1 mit dem Unterschied, daß man statt 26,8 g (0,4 Mol) Allylcyanid 30,45 g (0,3 Mol) 3-Chlor-but-3-ennitril einsetzt. Man erhält 36,1 g eines Rohprodukts, das laut GC/MS-Analyse zu 75,6 % (entsprechend 44,6 % der Theorie) aus 2,4-Dichlor-3,5-pyridindicarboxaldehyd besteht. Nach dem Umkristallisieren aus Cyclohexan erhält man ein Reinprodukt vom Schmelzpunkt 89 bis 90°C.

Im Rohprodukt konnten durch GC/MS-Analyse 2,9 % 2,4-Dichlor-5-pyridincarboxaldehyd und 7,8 % 2,4-Dichlor-3-pyridincarboxaldehyd nachgewiesen werden. Geringerer Überschuß an Vilsmeier-Reagens erhöht den relativen Anteil dieser beiden Monoaldehyde. 2,4-Dichlorpyridin wird in keinem Fall nachgewiesen.

| ¹H-NMR (CD₃-CN) | |
|---|---|
| H | δ (ppm) |
| H an C-6 | 8,87 (s) |
| CHO an C-3 | 10,45 (s) |
| CHO an C-5 | 10,40 (s) |

### Beispiel 11

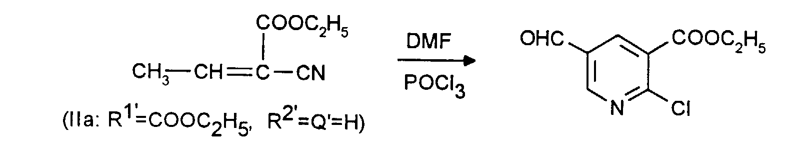

Man verfährt analog Beispiel 1 mit dem Unterschied, daß man statt 26,8 g (0,4 Mol) Allylcyanid 75,2 g (0,5 Mol) 92,6 %igen, destillierten Ethylidencyanessigsäureethylester einsetzt. Man erhält 60,0 g eines Rohprodukts, das durch fraktionierte Vakuumdestillation gereinigt wird. Der Hauptlauf siedet bei 122°C / 0,07 mbar. GC-Reinheit 93 %, Ausbeute 32,7 g (entsprechend 28,5 % der Theorie, bezogen auf 100 %ige Reinheit).

| ¹H-NMR (CDCl₃) | |
|---|---|
| H | δ (ppm) |
| H an C-4 | 8,47 (d) |
| H an C-6 | 8,85 (d) |
| CHO | 10,05 (s) |
| COOCH₂CH₃ | 4,33 (q) |
| COOCH₂CH₃ | 1,32 (t) |

### Beispiel 12

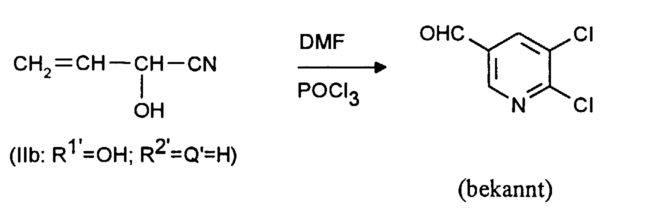

Man verfährt analog Beispiel 1 mit dem Unterschied, daß man statt 26,8 g (0,4 Mol) Allylcyanid 41,5 g (0,5 Mol) 2-Hydroxy-but-3-ennitril einsetzt. Man erhält 46,6 g eines Rohprodukts, das nach GC/MS-Analyse zu 75,7 % (entsprechend 40,1 % der Theorie) aus 2,3-Dichlor-5-pyridincarboxaldehyd besteht. Nach dem Umkristallisieren aus Cyclohexan farblose Nadeln vom Schmelzpunkt 68°C.

| ¹H-NMR (CDCl₃) | |
|---|---|
| H | δ (ppm) |
| H an C-4 | 8,26 (d) |
| H an C-6 | 8,78 (d) |
| CHO | 10,10 (s) |

Im Rohprodukt werden durch GC/MS-Analyse 15,3 % 2,5-Dichlor-3-pyridincarboxaldehyd nachgewiesen. Diese Verbindung ist nach dem Schema 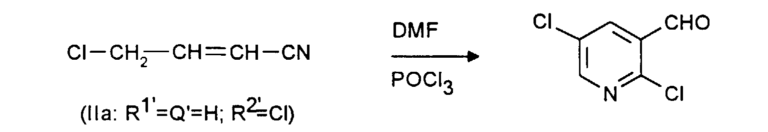 aus 4-Chlor-but-2-ennitril entstanden, das neben 2-Chlor-but-3-ennitril bekanntlich aus 2-Hydroxy-but-3-ennitril (= Cyanhydrin des Acroleins) unter der Einwirkung von Vilsmeier-Reagens gebildet wird (vergleiche J. Org. Chem. 55, (1990), 571).

### Beispiel 13

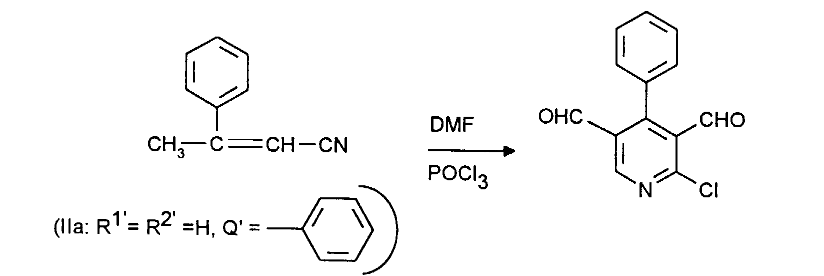

Man verfährt zunächst analog Beispiel 1 mit dem Unterschied, daß man statt 26,8 g (0,4 Mol) Allylcyanid 28,6 g (0,2 Mol) 3-Phenyl-but-2-ennitril einsetzt. Nach der Zugabe der DMF-Nitril-Mischung wird im Verlauf von 3 Stunden bis auf eine Endtemperatur von etwa 160°C hochgeheizt, dann auf Raumtemperatur abgekühlt und analog Beispiel 1 aufgearbeitet. Man erhält 33,2 g eines Reaktionsgemischs, in dem außer Ausgangsprodukt u.a. folgende Verbindungen nachgewiesen wurden:
2-Chlor-4-phenyl-3 -pyridincarboxaldehyd,
2-Chlor-4-phenyl-5-pyridincarboxaldehyd und
2-Chlor-4-phenyl-3,5-pyridindicarboxaldehyd.

### Beispiel 14

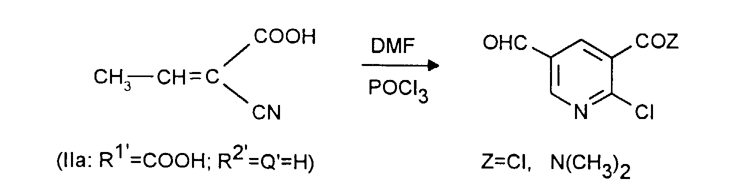

Zu 307 g (2 Mol) Phosphoroxychlorid wird unter Rühren und unter Feuchtigkeitsausschluß unter Eiskühlung bei 0 bis 5°C im Verlauf von etwa 30 Minuten eine Lösung von 22,2 g (0,2 Mol) Ethylidencyanessigsäure in 29,2 g (0,4 Mol) DMF getropft. Anschließend wird während einer Stunde bis auf 100°C erhitzt und dort noch weitere 3 Stunden gehalten. Dann wird abgekühlt und das überschüssige POCl₃ im Vakuum bei einer Badtemperatur von 25°C abgezogen. Die weitere Aufarbeitung erfolgt analog Beispiel 1. Man erhält 21,6 g eines Öls, dessen zwei Hauptkomponenten nach der GC/MS-Analyse zu 25,7 % aus 2-Chlor-3-chlorcarbonyl-5-pyridincarboxaldehyd und zu 57,1 % aus 2-Chlor-3-N,N-dimethylaminocarbonyl-5-pyridincarboxaldehyd bestehen.

### Beispiel 15

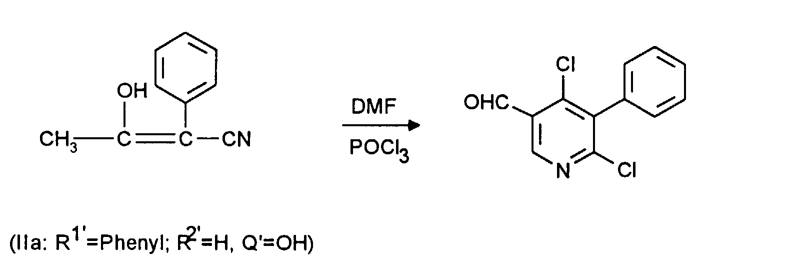

Man verfährt analog Beispiel 1 mit dem Unterschied, daß man statt 26,8 g (0,4 Mol) Allylcyanid 79,5 (0,394 Mol) 78,7 %iges 3-Hydroxy-2-phenyl-crotononitril einsetzt. Man erhält 35,0 g eines Rohprodukts, das laut GC/MS-Analyse zu 78,7 % (entsprechend 23,5 % der Theorie) aus 2,4-Dichlor-3-phenyl-5-pyridincarboxaldehyd besteht. Nach dem Umkristallisieren aus Cyclohexan schmilzt die Verbindung bei 106°C.

| ¹H-NMR (CDCl₃) | |
|---|---|
| H | δ (ppm) |
| H an C-6 | 8,86 (s) |
| CHO | 10,50 (s) |

### Beispiel 16

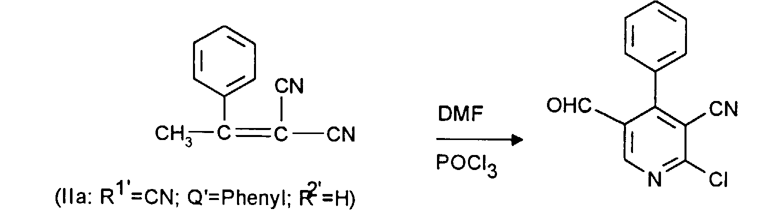

Zu 49 g (0,32 Mol) Phosphoroxychlorid wird unter Rühren und unter Feuchtigkeitsausschluß bei etwa 95°C im Verlauf von 15 Minuten eine Mischung aus 6,7 g (0,04 Mol)[1 -Phenyl-ethyliden]-malononitril und 23,4 g (0,32 Mol) DMF getropft. Es wird 24 Stunden bei 95 bis 100°C nachgerührt. Nach dem Abkühlen auf etwa 20°C wird analog Beispiel 1 aufgearbeitet. Man erhält 5,45 g Rohprodukt, das nach der GC/MS-Analyse 50,8 % 2-Chlor-3-cyan-4-phenyl-5-pyridincarboxaldehyd (entsprechend 28,5 % der Theorie) enthält. Nach Sublimation bei 150°C/0,1 mbar erhält man ein farbloses Sublimat, das nach dem Umkristallisieren aus Cyclohexan bei 152 bis 153°C schmilzt und 97,1 %igen oben genannten Aldehyd darstellt.

| ¹H-NMR (CDCl₃) | |
|---|---|
| H | δ (ppm) |
| H an C-6 | 9,09 (s) |
| CHO | 9,84 (s) |

### Beispiel 17

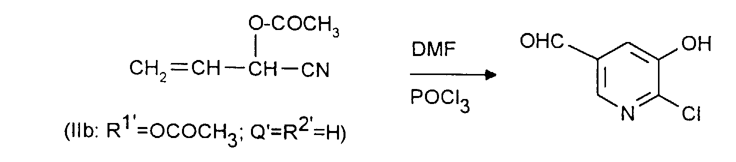

Man verfährt zunächst analog Beispiel 1 mit dem Unterschied, daß man statt 26,8 g (0,4 Mol) Allylcyanid 62,5 g (0,5 Mol) 2-Acetoxy-but-3-ennitril einsetzt. Nach dem Abkühlen auf etwa 20°C wird zuerst mit 500 ml Essigsäureethylester verrührt und anschließend 500 ml Wasser unter Kühlung so zugegeben, daß die Innentemperatur etwa 25°C nicht übersteigt. Anschließend wird portionsweise unter Rühren mit so viel festem Natriumacetat versetzt, bis ein pH-Wert von etwa 5 erreicht ist. Die organische Phase wird abgetrennt und die wäßrige Phase noch dreimal mit je 250 ml Essigester extrahiert.

Die vereinigten Essigesterphasen werden zweimal mit je 500 ml Wasser gewaschen, über Natriumsulfat getrocknet und im Rotationsverdampfer bei 14 mbar bis zu einer Badtemperatur von 80°C eingeengt.

Es hinterbleibt roher 2-Chlor-3-hydroxy-5-pyridincarboxaldehyd. Die Verbindung kann z.B. aus Toluol umkristallisiert werden und ist bei ca. 120°C / 0,1 mbar reinweiß sublimierbar. Sie färbt sich ab 185°C dunkel und ist bei 260°C noch nicht geschmolzen.

| ¹H-NMR (d₆-DMSO): | |
|---|---|
| H | δ (ppm) |
| H an C-4 | 7,67 (d) |
| H an C-6 | 8,42 (d) |
| CHO | 10,05 (s) |
| OH | 11,3 (s) |

### Beispiel 18

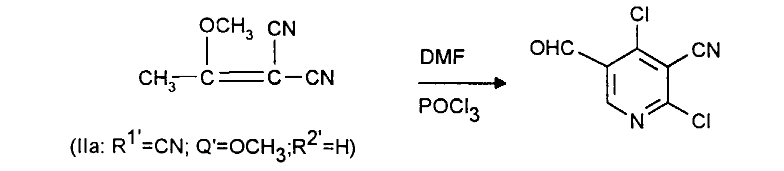

Man verfährt analog Beispiel 1 mit dem Unterschied, daß man statt 26,8 g (0,4 Mol) Allylcyanid 61 g (0,5 Mol) (1-Methoxyethyliden)-malononitril einsetzt. Man erhält 59,7 g eines Reaktionsgemisches, in dem durch GC/MS-Analyse 79,7 % 2,4-Dichlor-3-cyan-5-pyridincarboxaldehyd nachgewiesen wurden.

Nach dem Umkristallisieren aus Cyclohexan erhält man reinen Aldehyd vom Schmelzpunkt 128,5-129°C.

| ¹H-NMR (CDCl₃) | |
|---|---|
| H | δ (ppm) |
| H an C-6 | 9,00 (s) |
| CHO | 10,45 (s) |

### Beispiel 19

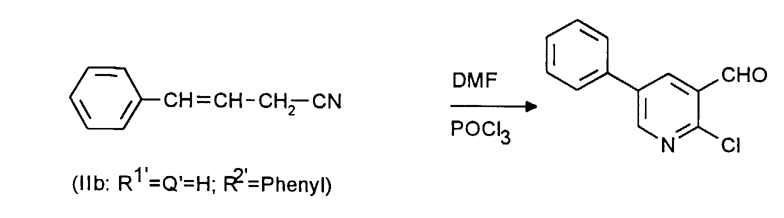

Zu 172 g (1,12 Mol) Phosphoroxychlorid wird unter Rühren und unter Feuchtigkeitsausschluß bei etwa 95°C im Verlauf von einer Stunde eine Mischung aus 40 g (0,28 Mol) 4-Phenyl-but-3-ennitril und 82 g (1,12 Mol) DMF getropft. Es wird 24 Stunden bei 95 bis 100°C nachgerührt. Nach dem Abkühlen auf etwa 20°C wird entsprechend Beispiel 1 aufgearbeitet. Man erhält 47,1 g 2-Chlor-5-phenyl-3-pyridincarboxaldehyd mit einer GC-Reinheit von 94,7 % (entsprechend 73,2 % der Theorie). Nach dem Umkristallisieren aus Petrolether schmilzt die Verbindung bei 87 bis 87,5°C.

| ¹H-NMR (CDCl₃) | |
|---|---|
| H | δ (ppm) |
| H an C-4 | 8,42 (d) |
| H an C-6 | 8,85 (d) |
| CHO | 10,51 (s) |

### Beispiel 20

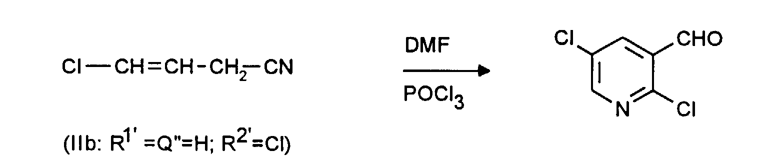

Man verfährt analog Beispiel 1 mit dem Unterschied, daß man statt 26,8 g (0,4 Mol) Allylcyanid 50,8 g (0,5 Mol) 4-Chlor-but-3-ennitril einsetzt. Man erhält 70,1 g (79 % der Theorie) 2,5-Dichlor-3-pyridincarboxaldehyd mit einer GC-Reinheit von 99,2 %. Die Verbindung ist bei 100°C / 0,1 mbar sublimierbar. Schmelzpunkt nach Umkristallisieren aus Cyclohexan 93 bis 94°C.

| ¹H-NMR (CDCl₃) | |
|---|---|
| H | δ (ppm) |
| H an C-4 | 8,20 (d) |
| H an C-6 | 8,60 (d) |
| CHO | 10,40 (s) |

### Beispiel 21

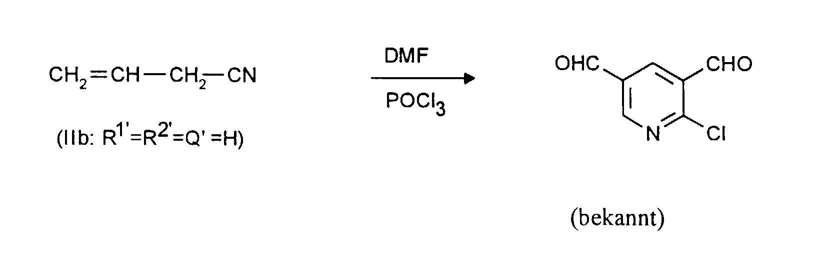

Man verfährt zunächst analog Beispiel 1. Die nach dem Ausschütteln mit Dichlormethan hinterbleibende wäßrige Phase wird unter Rühren und unter Kühlung (maximal 25°C) portionsweise mit so viel festem Natriumhydrogencarbonat versetzt, bis ein pH-Wert von 5 bis 6 erreicht ist. Danach wird noch dreimal mit je 250 ml Dichlormethan extrahiert und entsprechend Beispiel 1 aufgearbeitet. Man erhält so weitere 7,6 g laut GC 72,6 %igen 2-Chlor-3,5-pyridindicarboxaldehyd. Dadurch erhöht sich die Gesamtausbeute auf 58,5 % der Theorie.

### Beispiel 22

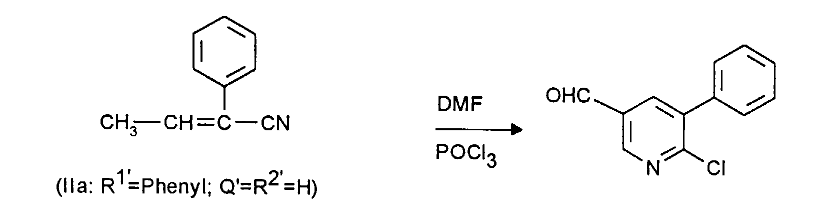

Man verfährt analog Beispiel 1 mit dem Unterschied, daß man statt 26,8 (0,4 Mol) Allylcyanid 71,5 g (0,375 Mol) 75 %iges 2-Phenyl-but-2-ennitril einsetzt. Man erhält 51,4 g eines Rohproduktes, das laut GC-MS-Analyse neben 37,7 % Ausgangsstoff 43,7 % 2-Chlor-3-phenyl-5-pyridincarboxaldehyd enthält. Das entspricht einer Ausbeute von 43,1 % der Theorie, bezogen auf umgesetzten Ausgangsstoff. Fraktionierte Destillation liefert bei 155 bis 160°C / 0,05 mbar das Pyridin in 85 %iger GC-Reinheit, das im Kühlschrank erstarrt. Nach dem Umkristallisieren aus Petrolether schmilzt die Verbindung bei 69°C.

| ¹H-NMR (CDCl₃) | |
|---|---|
| H | δ (ppm) |
| H an C-4 | 8,14 (d) |
| H an C-6 | 8,86 (d) |
| CHO | 10,15 (s) |

### Beispiel 23

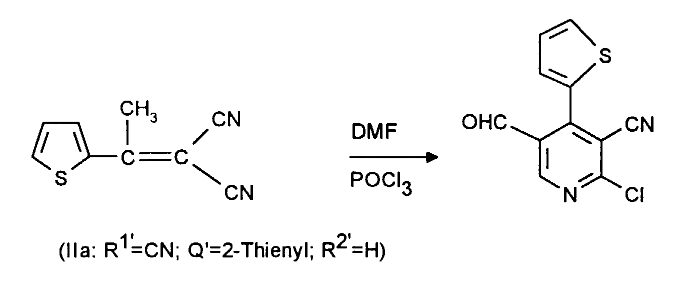

Man verfährt analog Beispiel 1 mit dem Unterschied, daß man statt 26,8 (0,4 Mol) Allylcyanid 43,5 g (0,25 Mol) [1-(2-Thienyl)ethyliden]-propandinitril einsetzt. Man erhält 40,4 g eines Rohproduktes, das laut GC-MS-Analyse zu 78,3 % (entsprechend 50,1 % der Theorie) aus 2-Chlor-3-cyan-4-(2-thienyl)-5-pyridincarboxaldehyd besteht. Nach dem Umkristallisieren aus Cyclohexan schmilzt die Verbindung bei 137,5 bis 138°C.

| ¹H-NMR (CDCl₃) | |
|---|---|
| H | δ(ppm) |
| H an C-6 | 9,03 (s) |
| CHO | 10,04 (s) |

## Patentansprüche

1. Verfahren zur Herstellung von Verbindungen der Formel (I) 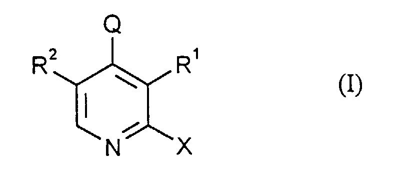 worin
X Chlor oder Brom,
Q Wasserstoff, Halogen, C₆-C₁₂-Aryl, Hetaryl mit 5 bis 7 Ringgliedern und 1 bis 3 Heteroatomen aus der Reihe N, O, S,
R¹, R² Formyl, Cyan, Hydroxyl, Halogen, C₁-C₈-Alkyl, Halogen-C₁-C₈-alkyl, Cyan-C₁-C₈-alkyl, C₁-C₄-Alkoxy-C₁-C₈-alkyl, C₃-C₁₀-Cycloalkyl, 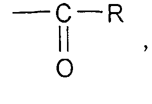 C₆-C₁₂-Aralkyl, C₆-C₁₂-Aryl, C₆-C₁₂-Aryloxy, Hetaryl mit 5 bis 7 Ringgliedern und 1 bis 3 Heteroatomen aus der Reihe N, O, S, Hetaryloxy mit 5 bis 7 Ringgliedern und 1 bis 3 Heteroatomen aus der Reihe N, O, S im Hetarylteil, mit der Maßgabe, daß mindestens einer der beiden Substituenten R¹ und R² für Formyl steht,
R Halogen, C₁-C₄-Alkoxy, Phenoxy, -NR'R" und
R', R" unabhängig voneinander C₁-C₄-Alkyl oder beide Substituenten zusammen C₄-C₆-Alkylen, das durch ein Sauerstoffatom unterbrochen sein kann, bedeuten,
dadurch gekennzeichnet, daß man eine Verbindung der Formel (II) 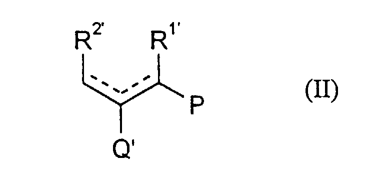 worin
die gestrichelte Linie die beiden möglichen Positionen einer C-C-Bindung anzeigt,
Q' die für Q definierten Bedeutungen annehmen kann oder Hydroxyl oder C₁-C₄-Alkoxy bedeutet,
P -CN, -CONH₂ oder -CH=NOH und
R^{1'}, R^{2'} unabhängig voneinander Wasserstoff, Cyan, Halogen, -CONH₂, C₁-C₈-Alkyl, Halogen-C₁-C₈-alkyl, Cyan-C₁-C₈-alkyl, C₁-C₄-Alkoxy-C₁-C₈-alkyl, Hydroxy-C₁-C₈-alkyl, C₁-C₈-Alkoxy, C₃-C₁₀-Cycloalkyl, 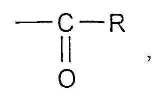 Carboxyl, C₁-C₈-,Acyloxy, C₆-C₁₂-Aralkyl, C₆-C₁₂-Aryl, C₆-C₁₂,-Aryloxy, Hetaryl mit 5 bis 7 Ringgliedern und 1 bis 3 Heteroatomen aus der Reihe N, O, S, Hetaryloxy mit 5 bis 7 Ringgliedern und 1 bis 3 Heteroatomen aus der Reihe N, O, S im Hetarylteil mit der Maßgabe, daß mindestens einer der beiden Substituenten R^{1'} und R^{2'} für Wasserstoff steht, bedeuten und
R die in Formel (I) angegebene Bedeutung besitzt, mit einem Vilsmeier-Reagens umsetzt und die Verbindungen I isoliert.

## Claims

1. Process for the preparation of compounds of the formula (I) 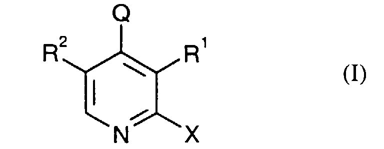 in which
X denotes chlorine or bromine,
Q denotes hydrogen, halogen, C₆-C₁₂-aryl, hetaryl having 5 to 7 ring members and 1 to 3 hetero atoms from the group consisting of N, O, S,
R¹, R² denote formyl, cyano, hydroxyl, halogen, C₁-C₈-alkyl, halogeno-C₁-C₈-alkyl, cyano-C₁-C₈-alkyl, C₁-C₄-alkoxy-C₁-C₈-alkyl, C₃-C₁₀-cycloalkyl, 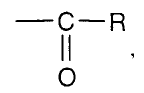 C₆-C₁₂-aralkyl, C₆-C₁₂-aryl, C₆-C₁₂-aryloxy, hetaryl having 5 to 7 ring members and I to 3 hetero atoms from the group consisting of N, O, S, hetaryloxy having 5 to 7 ring members and 1 to 3 hetero atoms from the group consisting of N, O, S in the hetaryl moiety, with the restriction that at least one of the two substituents R¹ and R² represents formyl,
R denotes halogen, C₁-C₄-alkoxy, phenoxy, -NR'R" and
R', R" denote, independently of each other, C₁-C₄-alkyl or both substituents together denote C₄-C₆-alkylene which can be interrupted by an oxygen atom,
characterized in that a compound of the formula (II) 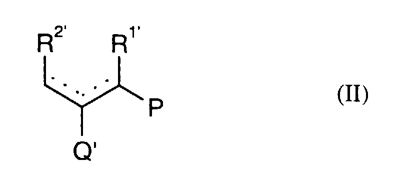 in which
the dashed line indicates the two possible positions of a C-C bond,
Q' can assume the meanings defined for Q or denotes hydroxyl or C₁-C₄-alkoxy,
P denotes -CN, -CONH₂ or -CH=NOH and
R^{1'}, R^{2'} denote, independently of each other, hydrogen, cyano, halogen, -CONH₂, C₁-C₈-alkyl, halogeno-C₁-C₈-alkyl, cyano-C₁-C₈-alkyl, C₁-C₄-alkoxy-C₁-C₈-alkyl, hydroxy-C₁-C₈-alkyl, C₁-C₈-alkoxy, C₃-C₁₀-cycloalkyl, 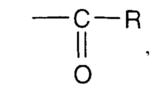 carboxyl, C₁-C₈-acyloxy, C₆-C₁₂-aralkyl, C₆-C₁₂-aryl, C₆-C₁₂-aryloxy, hetaryl having 5 to 7 ring members and 1 to 3 hetero atoms from the group consisting of N, O, S, hetaryloxy having 5 to 7 ring members and 1 to 3 hetero atoms from the group consisting of N, O, S in the hetaryl moiety with the restriction that at least one of the two substituents R^{1'} and R^{2'} represents hydrogen and
R has the meaning specified in formula (I)
is reacted with a Vilsmeier reagent and the compounds I are isolated.

## Revendications

1. Procédé de préparation des composés de formule I 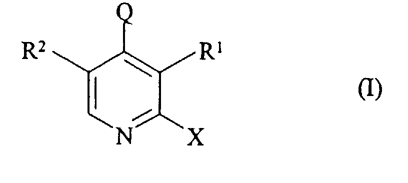 dans laquelle
X représente le chlore ou le brome,
Q représente l'hydrogène, un halogène, un groupe aryle en C₆-C₁₂, hétéroaryle de 5 à 7 chaînons cycliques contenant de 1 à 3 hétéroatomes choisis parmi N, O et S,
R¹ et R² représentent chacun un groupe formyle, cyano, hydroxy, un halogène, un groupe alkyle en C₁-C₈, halogénoalkyle en C₁-C₈, cyano-alkyle en C₁-C₈, (alcoxy en C₁-C₄) alkyle en C₁-C_{8,} cycloalkyle en C₃-C₈, 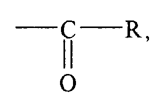 aralkyle en C₆-C₁₂, aryle en C₆-C₁₂, aryloxy en C₆-C₁₂, hétéroaryle de 5 à 7 chaînons cycliques contenant 1 à 3 hétéroatomes choisis parmi N, O et S, hétéroaryloxy de 5 à 7 chaînons cycliques et contenant 1 à 3 hétéroatomes choisis parmi N, O et S dans la partie hétéroaryle,
sous réserve que l'un au moins des deux symboles R¹ et R² représente un groupe formyle,
R représente un halogène, un groupe alcoxy en C₁-C₄, phénoxy, -NR'R" et
R' et R" représentent chacun, indépendamment l'un de l'autre, un groupe alkyle en C₁-C₄ ou forment ensemble un groupe alkylène en C₄-C₆ qui peut être interrompu par un atome d'oxygène,
caractérisé en ce que l'on fait réagir un composé de formule II 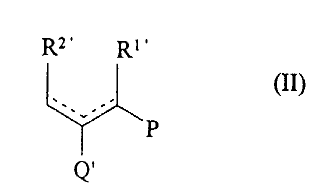 dans laquelle
les traits interrompus indiquent les deux positions possibles d'une liaison C-C,
Q' a les significations indiquées ci-dessus pour Q ou représente un groupe hydroxy ou alcoxy en C₁-C₄,
P représente -CN, -CONH₂ ou -CH=NOH et
R^{1'}, R^{2'} représentent chacun, indépendamment l'un de l'autre, l'hydrogène, un groupe cyano, un halogène, un groupe -CONH₂, alkyle en C₁-C₈, halogénoalkyle en C₁-C₈, cyano-alkyle en C₁-C₈, (alcoxy en C₁-C₄) alkyle en C₁-C₈, hydroxyalkyle en C₁-C₈, alcoxy en C₁-C₈, cycloalkyle en C₃-C₁₀, 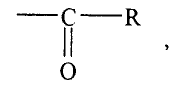 carboxyle, acyloxy en C₁ -C_{8,} aralkyle en C₆-C₁₂, aryle en C₆-C₁₂, aryloxy en C₆-C₁₂, hétéroaryle de 5 à 7 chaînons cycliques contenant 1 à 3 hétéroatomes choisis parmi N, O et S, hétéroaryloxy de 5 à 7 chaînons cycliques et contenant 1 à 3 hétéroatomes choisis parmi N, O et S dans la partie hétéroaryle, sous réserve que l'un au moins des deux symboles R^{1'} et R^{2'} représente l'hydrogène, et
R a les significations indiquées en référence à la formule I,
avec un réactif de Vilsmeier, et on isole les composés I.
